# EUROPEAN PATENT APPLICATION

(11) **EP 4 850 705 A1**
(43) Date of publication of application: **09.12.2026**
(21) Application number: 25181215.2
(22) Date of filing: 05.06.2025
(51) Int. Cl.: A61L 27/26, A61L 31/04, C08L 23/06

(54) **BIO-BASED POLYMER BLEND FOR PREPARING A MEDICAL DEVICE**

(71) Applicant: GaleMed Corporation, Yilan County 268 (TW)
(72) Inventor: WILLEMSEN, Thomas, 106 Taipei (TW); CHIANG HSU, Chia, Taipei (TW); YANG, Kuang, Taipei (TW); HUIQIANG, Gan, Taipei (TW); CHENGHUO, Wang, Taipei (TW); FEI, He, Taipei (TW)
(74) Representative: Manitz Finsterwald Patent- und Rechtsanwaltspartnerschaft mbB

(57) **Abstract**

A polymer blend for preparing a medical device is provided characterized in that, the polymer blend comprises a first thermoplastic polymer and a second thermoplastic polymer wherein the said polymers are derived from renewable sources and have biobased carbon contents of at least 20-90% and of at least 90-100% respectively. The first and the second thermoplastic polymer may be present in a weight ratio of about 1:10 to about 10:1. The bio-based polymer blends disclosed herein are sustainable, and are eco-friendly as they reduce the CO₂ footprint significantly.

## Description

### FIELD OF THE INVENTION

The present invention relates to polymer blends for preparing a medical device and in particular, to polymer blends comprising bio-based polymers derived from renewable sources. More particularly, the present invention relates to bio-based polymers for making medical devices including breathing circuits, breathing tubes and the likes.

### BACKGROUND

Medical devices play a crucial role in healthcare, enabling the safe delivery of therapeutic treatments, patient support, and diagnostic applications. For fabrication of these medical devices, the polymeric materials are chosen based on their versatility, weight, mode of functioning and ease of processing. Polymeric materials which are frequently used for this purpose include thermoplastic polymers such as polyethylene (PE), polypropylene (PP), polyvinyl chloride (PVC), polyurethane (PU), ethylene-vinyl acetate (EVA) copolymers and the likes. Most medical-grade polymers are generally derived from non-biological sources such as fossil fuels. Synthetic polymers derived from fossil fuels exhibit enhanced stability and inertness, making them resistant to physiological environments without compromising their functional efficacy.

Proper disposal of used medical devices is essential to prevent infection and ensure compliance with hygiene protocols. The disposal is specifically carried out by incineration of used devices which eliminates potential biological contaminants and reduces the risk of disease transmission. Medical devices composed of fossil fuel-derived polymers pose environmental concerns upon combustion. The incineration of such materials results in the release of carbon dioxide (CO₂) and other greenhouse gases, contributing to global warming. Additionally, incomplete combustion may generate carbon monoxide (CO) which is a toxic byproduct with adverse health effects. Hence, while these materials provide the technical properties needed in medical applications, their CO2 footprint is not ideal.

Biobased polymers derived from renewable biological sources are currently being preferred for their reduced CO₂ emissions associated with medical device manufacturing. Biobased polymers reduce the dependency on fossil fuels thereby helping in their conservation as the availability of fossil fuels is limited.

Hence, there is a need to develop biopolymers that can be used as one to one replacements of conventional polymers and have same physical, functional and mechanical properties as those of polymers derived from non-biological sources.

### SUMMARY

In one embodiment, a polymer blend for preparing a medical device is disclosed. The polymer blend comprises of a first thermoplastic polymer and a second thermoplastic polymer wherein the polymers have a biological origin and are extracted from renewable sources. The first and the second thermoplastic polymers in the polymer blend have a biobased carbon content of about 20-90% and about 90-100% respectively.

Preferred renewable sources include bagasse, sugar cane, corn starch or potato starch which are readily available. In another embodiment, a method for synthesis of the polymers from these sources is provided. Synthesis of polymers from these sources helps to bind biogenic carbon in the process which reduces the carbon footprint and makes these polymers eco-friendly. The method comprises subjecting the bagasse to microbial fermentation that results in the production of ethanol. Ethanol undergoes dehydration in the presence of a catalyst to produce ethylene. The ethylene is then polymerized under high pressure conditions to obtain the desired polymer.

In certain embodiments, the first and the second thermoplastic polymers are present in the polymer blend in a weight ratio of about 1:10 to about 10:1. The weight ratios of the first and the second thermoplastic polymers can be varied based on the desired mechanical properties in the polymer blend. In some embodiments, the weight ratio of the first and the second thermoplastic polymer may be about 1:4, about 1:6 or about 1:8.

In other embodiments, a method to prepare a medical device from the biobased polymer blend is provided. In preferred embodiments, the medical device is prepared by the process of extrusion. The method comprises providing a first and a second thermoplastic polymer in a weight ratio of about 1:10 to about 10:1; mixing the polymers at a temperature of about 25-50⁰C for 5-10 minutes to obtain a polymer blend; adding the polymer blend to an extrusion machine with a mold block and a die of desired shape; heating the polymer blend at about 150-250⁰C for 2-5 minutes to achieve a molten state; blowing an inert gas through the mold vents at 8-10 L/min while the polymer is being heated. Extruding the molten polymer blend through die. The blowing of air is continued to push the molten blend to reach mold block, molding the medical device upon shape of the mold block for a period of about 40-60 seconds; extruding the molten polymer blend through the mold block to form the medical device according to the shape of the mold block. The method further comprises the steps of cooling and annealing the extruded device. In preferred embodiments, the inert gas is blown continuously during melting of the polymer and molding process to remove odor.

A general description of the preferred embodiments and the detailed disclosure is provided which is intended to illustrate the principles of the invention and should not be construed as limiting in scope or its applicability.

### BRIEF DESCRIPTION OF THE DRAWINGS

A more complete appreciation of this disclosure and many of the associated advantages thereof will be readily obtained as the same becomes better understood by reference to the following detailed description when considered in connection with the accompanying drawings, wherein:
FIG. 1 illustrates an exemplary breathing tube 100.
FIG. 2 is a flowchart depicting a method of extraction of biobased polymers from bagasse.
FIG. 3 is a flowchart depicting a method of preparing a medical device from the biobased polymer blend of the present invention.
FIG. 4 shows Differential Scanning Calorimeter (DSC) analysis of biobased ethylene-vinyl acetate copolymer (EVA).
FIG. 5 shows Differential Scanning Calorimeter (DSC) analysis of biobased Low-density polyethylene (LDPE).
FIG. 6 shows Fourier-transform infrared spectroscopic (FTIR) analysis of biobased ethylene-vinyl acetate copolymer (EVA).
FIG. 7 shows Fourier-transform infrared spectroscopic (FTIR) analysis of biobased Low-density polyethylene (LDPE).

### DETAILED DESCRIPTION

In the provided drawings, reference numerals indicate same or related parts consistently across different views. Furthermore, the terms "a" and "an," unless explicitly defined otherwise, typically indicate "one or more. Furthermore, the terms "approximately," "approximate", "about" and similar terms generally refer to ranges that include the identified value within a margin of 20%, 10%, or preferably 5%, and any values therebetween.

The weight percent of a component is calculated based on the total weight of the formulation or composition in which it is present. For instance, if a component is specified as 5 wt. %, it means that this percentage is relative to a total composition of 100%.

As used herein, the term "polymer" refers to a larger molecule made of multiple smaller units of smaller molecules, repeating themselves in a specific arrangement.

As used herein, the term "bio-based" refers to materials, products, or fuels that are derived from natural sources, mainly plants, animals, or microorganisms.

As used herein, the term "polymer blends" refers to materials made by mixing two or more different polymers together having improved properties.

As used herein, the term "thermoplastic polymer" refers to a polymer that softens when heated and hardens when cooled. These polymers can be easily shaped, molded and recycled.

As used herein, the terms "biological sources", "renewable sources" and "sustainable sources" are used interchangeably and they collectively refer to sources that have a biological origin, are environment friendly and which naturally replenish over time.

As used herein, the term "molding" refers to the process of shaping a material into a specific form using a mold.

As used herein, the term "extrusion" refers to a process where a material, in molten state, is forced through a die to create objects with a continuous, uniform cross-section.

As used herein, the terms "gas" and "air" are used interchangeably and refer to any inert gas or dry air.

Disclosed herein are polymer blends comprising bio-based polymers wherein the bio-based polymers are extracted from sustainable sources. The bio-based polymer blends reduce overall carbon emissions and possess functional efficiency equivalent to that of polymers derived from non-renewable sources.

The bio-based polymers may be derived from any biological resource which is readily available. Suitable examples of biological resources include plant-based carbohydrates, by-products from microbial fermentation, agricultural products and their by-products including bagasse, sugar cane and the likes. Plant-based carbohydrates can be extracted from corns, potatoes, sugarcane and similar agricultural crops. Microbial fermentation products are sometimes used to produce biopolymers having desirable mechanical and thermal properties. The biological resource may be chosen based on its availability, cost-effectiveness and compatibility with the polymerization process. In an example, the bio-based polymers of the present invention are produced from bagasse which is abundantly available and cost-effective. Additionally, the use of bagasse for bio-based polymers helps in reducing the CO₂ emission since the sugarcane absorbs CO₂ when it grows and when a medical device formed from bio-based polymers is incinerated after use, this CO₂ is released back to the air. This greatly reduces the additional amount of CO₂ which is released after incineration of used medical devices based on polymers from fossil fuels.

In some embodiments, the polymer blends of the present invention comprise a first thermoplastic polymer and a second thermoplastic polymer derived from a biological resource. The biocarbon content in the first and second polymers may vary based on the desired mechanical properties of the polymer blend. In certain embodiments, the first thermoplastic polymer may have a biocarbon content of at least 20%-90%, at least 25%-85%, at least 30%-80%, at least 35%-75%, at least 40%-70%, at least 45%-65%, at least 50%-60%. In an example, the first thermoplastic polymer may have a biocarbon content of at least 55%. In another example, the first thermoplastic polymer may have a biocarbon content of at least 45%. In a preferred embodiment, the first thermoplastic polymer may have a biocarbon content of at least 80%.

In other embodiments, the second thermoplastic polymer may have a biocarbon content of at least 90%-100%, at least 92%-98% or at least 94%-96%. In an example, the second thermoplastic polymer may have a biocarbon content of at least 95%.

The polymers for the polymer blend may be chosen based on their biocompatibility and durability. Additionally, the combination of first and second polymers must result in a lightweight, chemically inert matrix with enhanced gas barrier properties, enabling efficient respiratory function. In such cases, polymers having a biological origin and in possession of these properties can be used in the polymer blends.

In an example, the bio-based polymer may be a thermoplastic polymer. In some embodiments, the first and the second thermoplastic polymers may be selected from a group consisting of Ethylene Vinyl acetate copolymer (EVA), Low-density polyethylene (LDPE), High-Density Polyethylene (HDPE), Polypropylene (PP) and combinations thereof. In a preferred embodiment, the first thermoplastic polymer may be Ethylene Vinyl acetate copolymer (EVA), Low-density polyethylene (LDPE) or High-Density Polyethylene (HDPE). In another preferred embodiment, the first thermoplastic polymer may be Ethylene Vinyl acetate copolymer (EVA).

In other embodiments, the second thermoplastic polymer may be selected from Low-density polyethylene (LDPE), High-Density Polyethylene (HDPE) and Polypropylene (PP). In an example, the second thermoplastic polymer may be Low-density polyethylene (LDPE).

In specific embodiments, the first thermoplastic polymer is different from the second thermoplastic polymer. For example, the polymer blends of the present invention may comprise Ethylene Vinyl acetate copolymer (EVA) as the first thermoplastic polymer and Low-density polyethylene (LDPE) as the second thermoplastic polymer.

The proportion of first thermoplastic polymer and the second thermoplastic polymer in the polymer blend may be varied to obtain a medical device with desirable properties. In certain embodiments, the first thermoplastic polymer and the second thermoplastic polymer may be present in a weight ratio of about 1:10 to about 10:1. As the first thermoplastic polymer will have different properties than the second thermoplastic polymer, the weight of the first or the second polymer may be increased or decreased based on the type, usage and desired properties of the device. Accordingly, the first and second thermoplastic polymers may be taken in a weight ratio of about 2:9, about 3:8, about 4:7, about 5:6, about 6:5, about 7:4, about 8:3 or about 9:2. In exemplary embodiments, wherein the first and the second thermoplastic polymers are EVA and LDPE respectively, a weight ratio of 1:6 may be preferred. This ratio will result in polymer blends with predominant properties of LDPE. Such polymer blends may be useful for preparing breathing circuits or anesthesia circuits where higher flexibility of the hose, moisture resistance and chemical resistance are critical for performance.

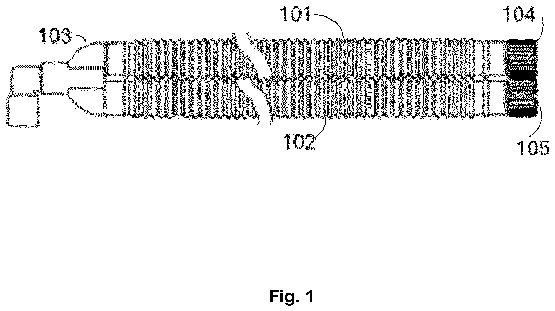

Referring to FIG. 1, an exemplary medical device **(100)** prepared from the polymer blends of the present invention, is illustrated. The medical device **(100)** depicts the tubing for an anesthesia breathing circuit. The tubing comprises dual hoses **(101, 102)** wherein the hose **(101)** is for inspiratory passage and the hose **(102)** is for expiratory passage. The hoses **(101, 102)** are connected at their proximal ends to a breathing mask via a Y-shaped connector **(103).** The distal ends **(104, 105)** of the hoses **(101, 102)** can be connected via two separate connectors to an anesthesia machine and a carbon dioxide scavenger machine respectively. The hoses are preferably ribbed along their length for a better grip and hold. The polymer blends herein can be used to prepare the entire anesthesia breathing circuit including the mask, hoses and the connectors.

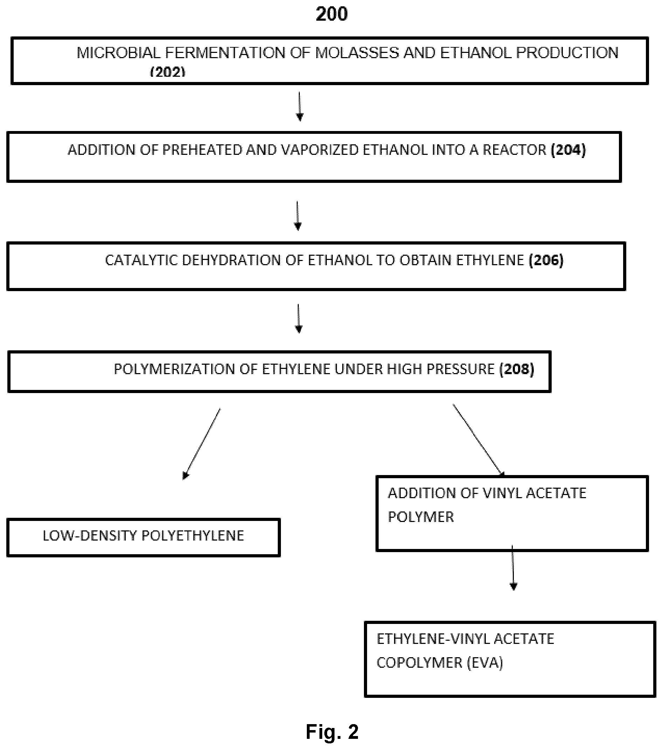

Referring to Fig. 2, a method 200 for the synthesis of the polymers from bagasse is illustrated. The steps in the method 200 may be executed in any order to achieve the intended result. Further, the steps may be added or removed without deviating from the intended scope of the disclosure.

At step 202, bagasse is subjected to fermentation by microbes. Bagasse is a by-product of sugarcane and contain fermentable sugars. Microbes including yeast and bacteria convert the fermentable sugars to ethanol. In specific embodiments, selected strains of yeast can be introduced into the fermentation medium at temperatures ranging from 28-32⁰C. The microbial strains are allowed to act on the medium for a period of 24-72 hours depending on the microbial strain and sugar concentration resulting into the production of ethanol.

At step 204, ethanol is preheated and vaporized and introduced into a reactor. This step is followed by the introduction of a catalyst to the reactor to ensure efficient contact with the catalyst. Suitable catalysts that can be used in the process may be solid acid catalysts that include gamma alumina, silica-based catalysts, alumina catalysts, zeolite and zirconia-based catalysts and the likes.

At step 206, ethanol is passed over the catalyst at high temperatures wherein hydroxyl groups of ethanol molecules are removed and ethylene is produced. In certain embodiments, ethanol is passed over the catalyst at temperatures of about 200°C to about 400°C. In specific embodiments, the temperature may be varied from about 250°C to about 350°C, preferably to about 300°C.

At step 208, polymerization of ethylene is carried out under high pressure to form LDPE. For production of EVA, vinyl acetate monomer (VAM) is incorporated into the polymerization reaction, creating an EVA copolymer. The content of vinyl acetate monomer (VAM) in EVA may be varied from 5-40 wt% to obtain a copolymer with desired elasticity and durability. For example, the EVA polymer having vinyl acetate at about 5-10 wt% will exhibit properties closer to those of polyethylene such as higher tensile strength, flexibility and durability. As the vinyl acetate weight % is increased, the EVA copolymer starts exhibiting properties of vinyl acetate including enhanced thermal resistance and a higher elongation at break.

The polymer blends of the present invention can be used to prepare medical devices including breathing circuits, anesthesia circuits, anesthesia (Airsoft) masks, non- invasive masks, heated wire circuits, a flex hose and connectors, oxygen or aerosol therapy products, nasal canulae, aerosol masks or gas sampling lines. In certain embodiments, the medical device can be prepared by the process of extrusion molding. In such cases, a general process of extrusion can be carried out in accordance with the state-of-the-art processes or as per industry standards.

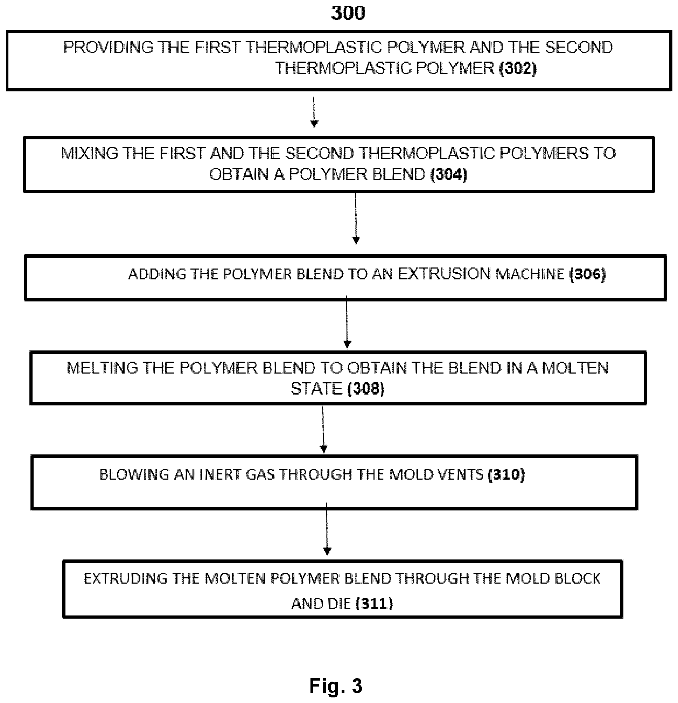

In an exemplary embodiment, a method 300 for preparing a medical device by extrusion of the polymer blends is provided. The method 300 is illustrated in Fig. 3 and the steps in the method 300 may be executed in any order to achieve the intended result. Further, the steps may be added or removed without deviating from the intended scope of the disclosure.

At step 302, the method 300 comprises providing the first thermoplastic polymer and the second thermoplastic polymer in a weight ratio of about 1:10 to about 10:1. In an exemplary embodiment, the first thermoplastic polymer and the second thermoplastic polymer are taken in a weight ratio of about 1:6.

At step 304, the method 300 comprises mixing the first and the second thermoplastic polymers to obtain a polymer blend. The polymers may be mixed manually or in a blender. Manual mixing may be performed by using a stirrer. In preferred embodiments, the polymers are mixed using a blender to achieve blends with a uniform distribution of components. The mixing may be carried out at temperatures of about 25-50⁰C wherein specific temperatures of about 30-40⁰C may be preferred. In certain embodiments, the mixing may be carried out at temperatures of about 40⁰C for a period of about 5-10 minutes. In other embodiments, the mixing may be carried out for a period of about 7-8 minutes or for a period till a smooth blend is obtained.

Once the polymer blend is prepared, the blend is fed into an extrusion machine for molding. Any extrusion machine known in the art can be used for extrusion molding of the polymer blend. An extrusion machine with a vent for blowing air inside is preferred. Accordingly, at step 306, the polymer blend is fed into the extrusion machine.

At step 308, the method 300 comprises melting the polymer blend at relatively higher temperatures to obtain the blend in a molten state. The polymer blend may be heated at temperatures of about 150-200⁰C for upto 5 minutes. In some embodiments, the polymer blend may be heated at about 155°C, about 160°C, about 165°C, about 170°C, about 175°C, about 180°C, about 185°C or at about 190°C. The heating may be continued for a period of 2 minutes, 3 minutes, 4 minutes or 5 minutes for proper melting of the blend.

To obtain an odor free product, the method 300 includes step 310 wherein an inert gas is blown through a mold vent while heating of polymer blend is being carried out. The inert gas may be selected from nitrogen or dry air. The inert gas is blown at a flow rate of about 8-10L/min during heating and melting of the polymer blend. The extrusion machine may be fitted with a mold block and a die of specific shape and size to obtain a medical device of desired dimensions and weight. For example, when the medical device is a breathing circuit, the hose of the circuit may have a length of about 1.5m and may have a weight of about 120gm to about 130gm. In such a case, the mold block and die may be chosen accordingly.

At step 311, the method 300 includes extruding the molten polymer blend through a die. The blowing of air is continued till the molten blend is pushed through the die, to reach the mold block. The final extruded product is the medical device that attains the desired shape while being extruded from the mold block.

The step 311 may be followed by cooling and annealing of the extruded device wherein the extruded device is cooled for a period of about 50-60 seconds or for a period till the temperature of the molded device reaches to about 20⁰C -30°C, preferably 25-30⁰C. The extruded device is then annealed for a period of about 15 hours, 20 hours or 25 hours at a temperature of about 20⁰C-30⁰C to reduce internal stresses in the final product. In a preferred embodiment, the device may be annealed at a temperature of about 25°C. The air is blown continuously during the extrusion molding process for a period of about 50-60 seconds to form the final odor free product. A sensory odor testing is performed on the final product to ensure that the device does not have any specific odour.

The polymer blends according to the present invention exhibit a tensile strength in the range of 160 kg/ cm² to about 220 kg/ cm². Further, the polymer blend exhibits a Vicat softening point of 77°C to about 96°C and a melt flow index of about 2.3 to about 3.0.

The airway resistance and flow resistance values upon bending, of the medical device prepared herein, fall within the standard range specified by the ISO. In an example, an anesthesia circuit prepared from the present polymer blends exhibits an inspiratory and expiratory airway resistance of about 0.01 cmH₂O/l/min. Further, the flow resistance of the medical device upon bending is less than 150%. In an exemplary embodiment, the flow resistance upon bending for both inspiratory and expiratory hose is about 105%.

### EXAMPLES

The examples described herein are presented for illustrative purposes only and shall not be interpreted as limiting the scope of the present disclosure. Various modifications, adaptations, and alternative implementations may be made without departing from the fundamental principles and intended scope of this disclosure.

### Example 1: Characterization of biobased polymers

EVA and LDPE were synthesized from bagasse. Different polymer blend samples viz. M10-00012, M10-00202, M10-0011, M10-00201 were prepared with different proprotions of EVA and LDPE. The samples were characterized using Differential scanning calorimetry (DSC) and Fourier transform infrared spectroscopy (FTIR).

### Differential scanning calorimetry (DSC):

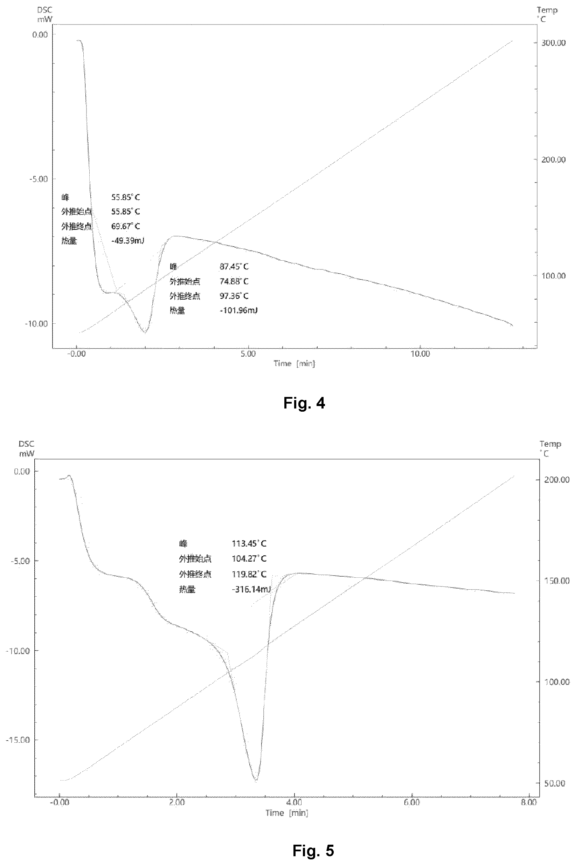

ISO 11357.1-2016 testing standard was followed. The samples were fed into a Differential scanning calorimeter (DSC214F) and heated from 20⁰C to about 200°C in the presence of nitrogen at a rate of 20⁰C/min. Fig. 4 illustrates the DSC spectrogram for EVA samples. The EVA samples M10-0011 and M10-00201 show melting points at 100.2⁰C and and 87.5⁰C respectively while the DSC spectrogram for the LDPE samples M10-00012 and M10-00202 as depicted in Fig. 5 shows the melting points at 115°C and and 113.5⁰C respectively.

### Fourier transform infrared spectroscopy (FTIR):

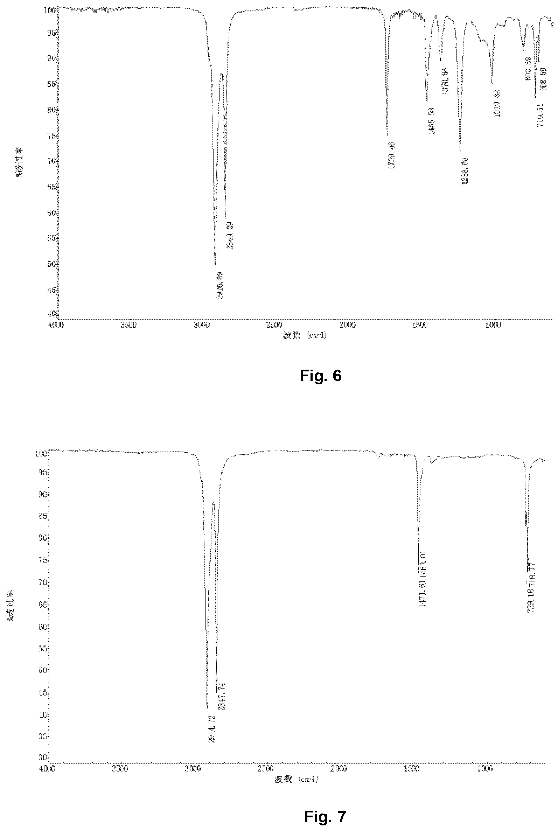

For FTIR, GB/T 6040-2019 testing standard was followed. Fig. 6 shows the FTIR spectra for EVA samples M10-0011 and M10-00201. The spectra show the absorption bands near 2847-2915 cm⁻¹ which correspond to the vibration absorption peaks of saturated C-H bonds. The FTIR spectra for LDPE samples M10-00012 and M10-00202 are illustrated in Fig. 7. In the FTIR spectra, the absorption bands are again seen near 2847-2915 cm⁻¹ which correspond to the C-H stretching in polyethylene chains.

### Example 2: Test protocol for Eco Anesthesia Circuit Adult, 1.5m (AB0437)

Ten Eco Anesthesia circuits were prepared from the biobased polymer blends and were tested on various aspects including appearance, weight, length, leakage, airway resistance, flow resistance upon bending, compliance, tensile strength and reliability. The ciruit samples were tested according to ISO 5367:2023 requirement standards.

### Preparation:

The temperature and humidity of the laboratory were kept within 23±3°C & 55 ± 20%RH. All the testing equipments were set within the calibration period. It was ensured that the samples do not contain hair, dirt, or foreign matter.

### Product Net Weight:

For weighing the sample circuits, Electronic scale (No.: CN01-25200136, Valid Period: 2024-12-17) was used and for reference, the net weight of each sample (including PE bag) was measured.

### Length measurement:

The breathing tube samples were placed on a horizontal plane and their lengths were measured under stationary conditions, expressed in meters.

### Leakage Test:

Leakage tester (No.: CN01-27900277, Valid Period: 2024-12-12) and Flow meter (No.: CN01-15200050, Valid Period: 2024-12-12) were calibrated according to ISO 5367:2023, Section 6.4.2 and Annex E. The samples were conditioned at a temperature of (23 ± 3) °C for at least 1 h prior to testing and the said temperature was maintained during testing. This was followed by applying and maintaining an internal gas pressure of (60±3) cmH₂O, the flow rate of air required to maintain the internal pressure was recorded. The leakage rate of the internal gas was tested and confirmed to not exceed 70 ml/min.

### Resistance Test:

The samples were conditioned at (23±3)°C for at least 1h prior to testing, then the same temperature was maintained during test. Without equipping the tube, the air flowrate was adjusted to 30l /min. until the value became stable. Then, the initial pressure value (P1) was recorded. With the tube equipped, the same flowrate of 30 l/min was maintained and the pressure value (P2) was recorded. The resistance value of tube was calculated as (P2-P1)/30 and the reference value for the resistance was set to not exceed 0.06cmH₂O.

### Increase in flow resistance with bending test:

ISO 5367:2023, section 6.5.3 and Annex G standards were followed. An air flow 30LPM was applied to the sample tube and the pressure of the tube held straight as P3 was recorded as shown in Certifier FA Plus. The breathing set was conditioned at (42 ±3)⁰C and at not less than 80 % relative humidity for at least 1 h. The test procedure was carried out at a temperature of (23 ± 3) ⁰C within 5 min after conditioning. The machine end of the tube was suspended over the cylinder to maintain the tube in continuous over half of the circumference of the cylinder. Then the air flow 30 LPM was applied to the tube and the air-pressure (P4) was recorded as shown in Certifier FA Plus at 5 min. The bending resistance values P3/P4 values were recorded and the increase in flow resistance with bending was set to not exceed 150% of the resistance.

### Compliance Test:

All leaks were sealed and retested until leakage was <1ml/min. The tube was tested at (42±3)⁰C and at not less than 80% relative humidity for at least 1h. The test procedure was conducted at (23±3)⁰C within 5 min after conditioning. The patient connection port was blocked and the tube was mounted in such a manner so as not to impede movement. The pressure-measuring device was connected to the open end of the tube and the test piece was inflated over a period of 5s with sufficient air to achieve a stable gauge pressure of (60±3) cmH₂O and the volume of air required was recorded. The breathing tube Compliance was set to not exceed 5 ml/cmH₂O. Reliability test:

Temperature & Humidity chamber (No.: CN01-15400025) were set according to ISO 10651-4:2002 A.4.13 and ASTM D4169-16 Schedule H Level 2 and the above steps were repeated.

### Table 1: (Table 6 under section Test Criteria)

### Example 3: Test results for Eco Anesthesia Circuit Adult, 1.5m (AB0437)

### Table 2 and Table 3 from sections 7.1 and 7.2

### Conclusion:

In the routine test, the Eco Anesthesia Circuit, Adult, 1.5m (P/N: AB0437) can meet ISO 5367:2023 requirement. After the reliability test, the Eco Anesthesia Circuit, Adult, 1.5m (P/N: AB0437) can meet ISO 5367:2023 requirement.

## Claims

1. A bio-based polymer blend to prepare a medical device comprising a first thermoplastic polymer and a second thermoplastic polymer wherein the first thermoplastic polymer includes a bio-based carbon content of at least 20-90% and the second thermoplastic includes a bio-based carbon content of at least 90-100% and,
wherein, the first thermoplastic polymer and the second thermoplastic polymer are present in a weight ratio of about 1:10 to about 10:1.

2. The bio-based polymer blend to prepare a medical device according to claim 1, wherein, the first thermoplastic polymer is selected from a group consisting of Ethylene Vinyl acetate copolymer (EVA), Low-density polyethylene (LDPE), High-Density Polyethylene (HDPE), Polypropylene (PP) and combinations thereof.

3. The bio-based polymer blend to prepare a medical device according to claim 1, wherein, the second thermoplastic polymer is different from the first thermoplastic polymer and is selected from a group consisting of Ethylene Vinyl acetate copolymer (EVA), Low-density polyethylene (LDPE), High-Density Polyethylene (HDPE), Polypropylene (PP) and combinations thereof.

4. The bio-based polymer blend to prepare a medical device according to any of claims 1-2, wherein the first thermoplastic polymer is ethylene-vinyl acetate copolymer (EVA) and wherein the first thermoplastic polymer exhibits a bio-based carbon content of at least 45%.

5. The bio-based polymer blend to prepare a medical device according to any of claims 1-3, wherein the second thermoplastic polymer is low-density polyethylene (LDPE) and wherein the second thermoplastic polymer exhibits a bio-based carbon content of at least 95%.

6. The bio-based polymer blend to prepare a medical device according to any of claims 4-5, wherein the first thermoplastic polymer and the second thermoplastic polymer are present in a weight ratio of about 1:6.

7. The bio-based polymer blend to prepare a medical device according to any of the above claims, wherein the EVA comprises 5-40 wt% vinyl acetate monomers.

8. The bio-based polymer blend to prepare a medical device according to any of the above claims, wherein the polymer blend has a tensile strength in the range of 160 kg/cm² to about 220 kg/cm².

9. The bio-based polymer blend to prepare a medical device according to any of the above claims, wherein the polymer blend exhibits a Vicat softening point of about 77°C to about 96°C

10. The bio-based polymer blend to prepare a medical device according to any of the above claims, wherein the polymer blend exhibits a melt flow index of about 2.3 to about 3.0.

11. A medical device comprising the bio-based polymer blend according to any of the above claims, wherein the medical device is a breathing circuit, an anaesthesia circuit, an anaesthesia (Airsoft) mask, non- invasive mask, heated wire circuit, a flexhose, a connector, an oxygen or aerosol therapy product, a nasal canula, an aerosol mask or a gas sampling line.

12. A medical device comprising the bio-based polymer blend according to any of the above claims, wherein the medical device is prepared by extrusion.

13. The medical device according to claims 11-12, wherein the medical device exhibits an airway resistance of less than 0.06 cmH₂O/l/min.

14. The medical device according to claims 11-12, wherein the flow resistance of the medical device upon bending is less than 150%.

15. A method of preparing a medical device from the bio-based polymer blend according any of the claims, comprising:
providing a first and a second thermoplastic polymer in a weight ratio of about 1:10 to about 10:1;
mixing the polymers at a temperature of about 25-50⁰C for 5-10 minutes to obtain a polymer blend;
adding the polymer blend to an extrusion machine with a mold block and a die of desired shape;
heating the polymer blend at about 150-250⁰C for 2-5 minutes to achieve a molten state;
blowing an inert gas through a mold vent at 8-10 L/min while the polymer is heated preferably for a period of about 40-60 seconds;
extruding the molten polymer blend through a die and a mold block to obtain the medical device.

16. The method according to claim 15, wherein the inert gas is blown continuously during the polymer melting and molding process to remove odour.

17. The method according to claim 15, wherein the inert gas is nitrogen or dry air.

18. The method of preparing a medical device according to claim 15, wherein the extruded device is cooled during molding process to reduce the temperature of the device to 30-50⁰C preferably for a period of about 50-60 seconds.

19. The method of preparing a medical device according to claim 15, further comprising annealing the extruded device at about 20⁰C-30⁰C for a period of 15-25 hours to reduce internal stresses.

20. The method of preparing a medical device according to any of the claims 15-19, wherein the medical device passes sensory odor testing with no detectable off-odors.
